# EUROPEAN PATENT SPECIFICATION

(11) **EP 0 301 584 B1**
(45) Date of publication and mention of the grant of the patent: **03.02.1993**
(21) Application number: 88112356.6
(22) Date of filing: 29.07.1988
(51) Int. Cl.: G01N 33/545, G01N 33/577, G01N 33/68

(54) **Immunological measuring method**
Immunologische Messmethode
Méthode de mesurage immunologique

(30) Priority: 31.07.1987 JP 190212/87
(43) Date of publication of application: 01.02.1989
(73) Proprietor: FUJIREBIO KABUSHIKI KAISHA, Tokyo 161 (JP)
(72) Inventor: Saito, Tomo, 4-chome, Shinyuku-ku, Tokyo (JP); Takeda, Hideo, 4-chome, Shinyuku-ku, Tokyo (JP); Sakurabayashi, Yasusuke, 4-chome, Shinyuku-ku, Tokyo (JP); Inouchi, Toshitsugu, 4-chome, Shinyuku-ku, Tokyo (JP); Suzuki, Norihiro, 4-chome, Shinyuku-ku, Tokyo (JP); Kikuchi, Yousuke, 4-chome, Shinyuku-ku, Tokyo (JP)
(74) Representative: Barz, Peter, Dr.

(56) References cited:
- EP-A- 0 106 685
- EP-A- 0 174 195
- JP-A- 5 281 566
- JP-A- 5 281 567
- JP-A-49 187 264
- JP-A-52 116 264
- US-A- 4 118 192
- CHEMICAL ABSTRACTS, vol. 106, no. 17, April 27, 1987, Columbus, Ohio(US); K. MITANI, page 329, column 2, abstract no. 134473x;
- CHEMICAL ABSTRACTS, vol. 104, no. 15, April 14, 1986, Columbus, Ohio(US); M. NOZAWA et al., page 361, column 2, abstract no. 126131c;
- CHEMICAL ABSTRACTS, vol. 102, no. 19, May 13, 1985, Columbus, Ohio (US); M.U. SUONPAA et al., page 308, column 1, abstract no. 163137p;

## Description

The present invention relates to an immunological measuring method in clinical test and, particularly, a high sensitivity immunological measuring method for antigen-antibody reaction utilizing fluorescent particles and an apparatus therefor.

A latex agglutination immunological measuring method utilizing latex constituting fluorescent particles which can be used in the present invention can be classified, generally, to the so-called slide glass method which is a qualitative immunological measuring method, a quantitative immunological measuring method in which an agglutination caused by an antigen-antibody reaction is picked up as a variation of turbidity which is optically measured by a nephelometer (cf. "Latex Photometric Immunoassay (LPIA)", Kensa To Gijutsu, Vol. 12, No. 7, pp. 581, 1984, JP-A- 24015/1978 and 187264/1984) and a method utilizing a phenomenon that two or more latex particles are agglutinated for antigen-antibody reaction in which the degree of agglutination of latex is numerically detected by a particle counter of electroresistive type or optical type (cf. " Particle Counting Immunoassay (PACIA)", J. of Immuno. Meth., Vol. 18, page 33, 1977, FCM, JP-A- 81567/1987).

The method which uses the nephelometer is advantageous over the enzyme immunoassay (EIA) in that the time necessary for reaction is short and there are highly automated apparatuses for performing it. However, since the sensitivity thereof related to immunological reaction is low compared with the EIA or RIA ( Radio Immunoassay ), it can not be used for detection of a minute sample which requires high sensitivity. Further, a measuring result obtained by the method using the nephelometer is disadvantageously affected by substances such as chylus serum and immunocomplex coexisting with a test sample. Although in order to eliminate this problem, there have been proposed methods such as a method using near infrared ray and the so-called rate method in which a variation rate of turbidity with time is measured, it is still insufficient to obtain an acceptable sensitivity for immunoassay.

The method using the particle counter is theoretically highly sensitive. However, also in this method, substances coexisting with the latex may cause noise. Further, since, in performing this method, it is necessary to flow a fluid through a very thin nozzle, there is a clogging problem of the nozzle by dust etc. In addition, since, in this method agglutination is measured while it is moving through the nozzle, there is a problem of decomposition of agglutinated substances.

EP-A-174195 discloses a method in which two or more insoluble coloured substances, each substance being adapted to form a distinctively coloured agglutinate in the presence of a specific ligand or group of ligands is determined by eye inspection of the agglutinates. However, this assay allows qualitative determinations only.

An object of the present invention is to provide a novel immunological measuring method using fluorescent latex particles which method allows fast and highly sensitive determinations. Another object of the invention is to provide an apparatus for performing said measuring method.

The immunological measuring method according to the present invention comprises the steps of:
a) preparing a fluorescent particle reagent carrying antibody or antigen and having a mean size of not more than 0.3 µm;
b) preparing a solid phase reagent carrying antibody or antigen;
c) pouring the fluorescent particle reagent and a sample which is suspected to contain an antigen or antibody corresponding to the antibodies or antigens of said reagents to the solid phase reagent and allowing an antigen-antibody reaction to occur; and
d) quantitatively measuring those fluorescent particles coupled to the solid phase reagent.

The apparatus according to the present invention which is suitable for carrying out said measuring method comprises:
- a flat-bottomed plate serving as a solid phase reagent carrying antibody or antigen at its surface;
- a fluorescent microscope;
- a TV camera for picking up a fluorescent particle image or a pattern of fluorescent particles through said fluorescent microscope; and
- an image processor for processing the fluorescent particle image or pattern of fluorescent particles obtained by said TV camera.

Fluorescent particles carrying antigen or antibody to be used in the present invention can be prepared according to the techniques disclosed in e.g. JP-A-81566/1987, 81567/1987 or 116264/1987. As latex of these fluorescent particles, other materials than polystyrene disclosed in the above disclosures, such as styrene butadiene copolymer or styrene-acrylic acid copolymer, may be used. As the fluorescent material, organic fluorescent material such as fluorescein or rhodamine, inorganic fluorescent material such as platinum cyanide or sulfates of alkaline earth metals or any combination thereof may be used. Any antigen or antibody may be attached to the particle. For example, antigens such as cancer marker or HBₛ or antibodies such as I_{g} G or I_{g} M may be used. These substances may be attached to the particle by physical adhesion or chemical reaction utilizing functional groups on the particle.

Further, it is possible to use minute crystals of fluorescent dye of naphthalimide or benzotriazole type directly as carriers for attaching antibody or antigen to latex particles.

The size of the fluorescent particle is not limited so long as the amount of fluorescent light emitted thereby is enough to be measured as a point light source. This is advantageous over the conventional agglutination measuring method using the particle counter which requires the use of latex having a size of around 1 µm in that particle size usable is in the order of 0.1 µm or even smaller which is detected by the conventional particle counter as noise, since the agglutination reaction has a higher speed with smaller particle size. However, when a centrifugal separation of fluorescent particles is employed, the size of the particle should be selected suitably, since it is impossible to apply the centrifugal separation to too small particles.

An apparatus for performing the present method comprises a fluorescent microscope, a TV camera associated therewith and an image processor.
In the drawing:
Fig. 1 shows an embodiment of an apparatus for performing the present method, in block;
Figs. 2 and 3 are graphs showing relations of concentration of AFP to total area of agglutination, respectively; and
Fig. 4 is a graph showing a relation of total number of adsorbed latex particles to absolute value of AFP.

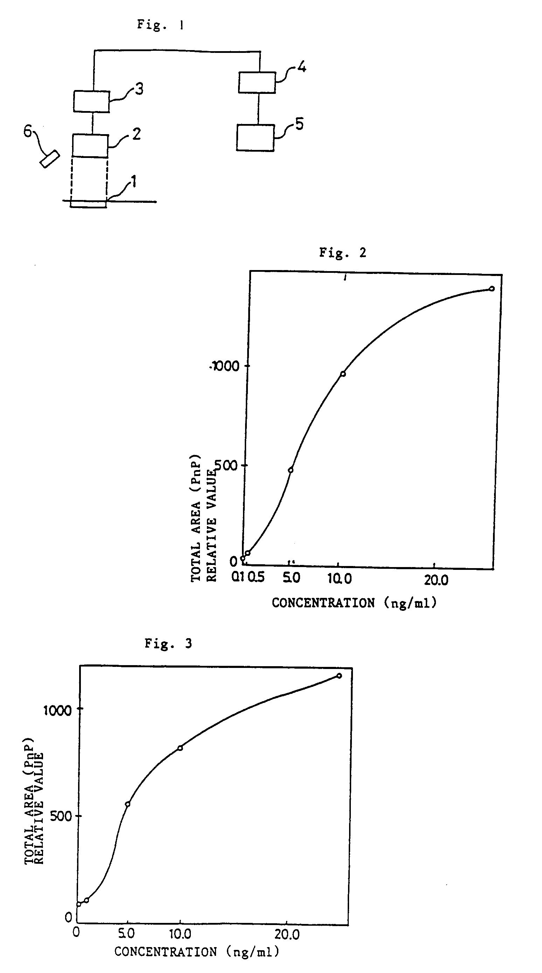

In Fig. 1 which is a block diagram of an apparatus for performing the present method, a fluorescent microscope 2 is disposed such that a TV camera 3 can pick up a fluorescent particle image in a cuvette 1 therethrough. A video image obtained by the TV camera 3 is processed by an image processor 4. It is preferable to use, additionally, a microcomputer 5 for digitizing the image, a ultra violet ray source such as high voltage mercury tube 6 emitting an exciting light, a monitor television (not shown) for displaying an output of the microcomputer and a printer (not shown) for printing the output.

The cuvette 1 is of a block or other colored material and has a depth as thin as about 1 mm. An image obtainable by the use of the cuvette 1 and the fluorescent microscope 2 may be changed time to time due to convection in the cuvette. However, with the use of the cuvette 1 having such depth, it is possible to pick up fluorescent particles therein with the fluorescent microscope 2 focussed thereto. Thus, an autofocus mechanism for automatically focussing the fluorescent microscope which is not commercially available can be omitted.

The wavelength and intensity of light from the light source 6 may be selected according to a fluorescent material to be contained in the particle.

The image processor 4 comprises a video input portion, a digital image memory portion for storing data of respective images and a data output portion for supplying the data to the computer on demand.

The image processing is based on a difference in fluorescent light intensity between fluorescent particles agglutinated and those not agglutinated. That is, particles which emit light not lower than a predetermined level are measured. The measurement is any of the counting of the particles, the measuring of an area formed by the particles and the calculation of mean particle size from the area and the number of the particles and a result is digitized by the microcomputer. A standard solution of antigen or antibody is used in an actual measurement and numerical values obtained at respective concentration of the solution are plotted to obtain detection lines.

### EXAMPLE 1 MEASUREMENT OF HUMAN AFP

### (1) PREPARATION OF REAGENT

Fluorescent polystyrene latex (Fluoresbrite®, registered trademark of Polyscience Co., 17151) having a mean particle size of 0.2µm) and anti-human AFP antibody (rabbit)(DAKO) were used and fluorescent latex attached with anti-human AFP antibody was prepared according to the method of Bernard et al (Clin. Chem., 27(6), 832 (1981)).

### (2) FORMATION OF DETECTION LINE

A suspension of the resultant fluorescent latex of 10µℓ was added to 20µℓ of each of standard solutions containing human AFP control solution of different concentrations (Nippon Biotest Kenkyusho) and was agitated at 40°C for 30 minutes. Then, 2mℓ of saline solution containing 5 % Tween® 20 was added thereto and agitated. 70µℓ of the resultant solution was taken in the cuvette 1 and the latter was set under the fluorescent microscope 2. An image obtained by the TV camera 3 through the fluorescent microscope 2 was processed by the microcomputer 4 and a result was plotted. Fig. 2 shows detection lines obtained in this manner. In Fig. 2, an ordinate is a sum of areas of agglutinations (PnP) each including two or more latex particles are agglutinated.

### EXAMPLE 2 MEASUREMENT OF HUMAN AFP

The same operations as those in the Example 1 were performed, except that fluorescent polystyrene latex was Fluoresbrite® 17153 having a mean particle size of 0.75 µm, and detection lines shown in Fig. 3 were obtained.

From Figs. 2 and 3, it is clear that, according to the present method, it is possible to measure antigen or antibody of as small amount as 250 pg/mℓ which is highly sensitive compared with the conventional nephelometric measuring method which can measure in the order of 10 ng/mℓ at most.

In the above described embodiment, the antigen-antibody reaction occurs in the solution in the very shallow cuvette. In order to further improve this method, it is necessary to make the depth of the cuvette as small as possible. According to a second aspect of the present invention, antigen or antibody is fixed on a surface to form a solid phase and a suspension containing antibody or antigen attached to fluorescent latex particles is poured onto the solid phase antigen or antibody, so that a reaction occurs in substantially a plane. A microplate of polystyrene having a flat bottom, which is used for ELISA or EIA, or a microplate used for HLA typing may be suitable for the solid phase used in this invention.

An image of the cuvette obtained through the fluorescent microscope includes fluorescent latex coupled to the bottom of the microplate and latex floating in the solution near the solid phase while not coupled thereto. When the cuvette is of transparent material, it is possible to obtain the former latex efficiently by adding back pigment to the solution after the reaction completes.

The latter latex can be removed by obtaining a plurality of images sequentially and picking a latex image containing latex particles existing in all of the images. This is because that non-coupled latex particles move freely while coupled particles are not. Thus, it is possible to pick up only the latex image composed of the coupled latex particles by averaging luminance of each pixel of the data of the plurality of the images and, by plotting a relation between the number of the coupled latex particles and the titer of the immunological component, a detection line can be obtained.

### EXAMPLE-3

### (1) PREPARATION OF LATEX REAGENT HAVING ANTI-HUMAN α-FETOPROTEIN MONOCLONAL ANTIBODY

1 mℓ of Tris buffer solution (tris(hydroxymethyl) aminomethane buffer solution) of anti-human α- fetoprotein (AFP) monoclonal antibody (antibody concentration:100 µg/mℓ ) was mixed with 100µℓ of fluorescent polystyrene latex (Polyscience Co., concentration of solid phase component: 2.5 wt%) having a mean particle size of 0.3 µm and the mixture was agitated at 37°C for one hour and then centrifugally separated (12,000 rpm, 30 minutes).

Latex attached with anti-AFP monoclonal antibody and separated as above was washed three times with tris buffer solution containing 1.0 wt% bovine serum albumin such that a solution containing 0.002 wt% latex particles suspended is obtained.

### (2) PREPARATION OF PLATE REAGENT ATTACHED WITH ANTI-HUMAN α - FETOPROTEIN MONOCLONAL ANTIBODY

Phosphate buffer solution containing anti-human AFP monoclonal antibody having antigen recognition position different from that of anti-human AFP monoclonal antibody prepared in (1),(antibody concentration: 10 ug/mℓ ) was poured to a flat bottomed plate (Nunc Co.) having a plurality of wells by an amount of 50 uℓ /well and, after kept stationarily for 4 hours at room temperature, washed three times with phosphate buffered saline (Tw-PBS) containing 0.01 wt% Tween® 20.

Thereafter, phosphate buffer solution containing 1.0 wt% bovine serum albumin was added thereto by an amount of 100µℓ /well and, after kept stationarily at 4°C for one night, the mixture was washed three times with Tw-PBS, resulting in the aimed plate.

### (3) FORMATION OF STANDARD LINE

Tris buffer solution containing 1.0 wt% bovine serum albumin was added to the plate obtained in (2) by an amount of 10 µℓ /well and antigen-antibody reaction was allowed to occur at room temperature for one hour. Then, the latex prepared in (1) was added by an amount of 50µℓ /well and antigen-antibody reaction was allowed to occur at room temperature for 3 hours.

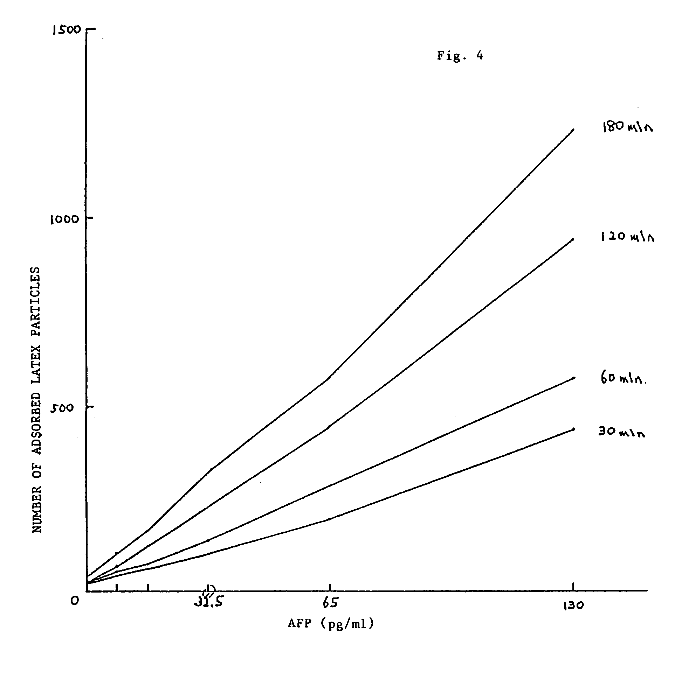

Thereafter, black ink was added by an amount of 200µℓ /well, and the plate was set to an inverted fluorescent microscope and an image was photographed with a high sensitive film (ISO 3200). Then, the number of latex particles adsorbed on the plate was counted and a standard curve was obtained. Fig. 4 shows the obtained standard curve together with other standard curves obtained by changing time for which the last antigen-antibody reaction occurs.

### EXAMPLE-4

The steps (1) and (2) in the Example-3 were used to prepare the latex reagent and the plate reagent. After antigen-antibody reaction for three hours in the step (3) in the Example-3, the plate was washed three times with phosphate buffered saline containing 0.01 wt% Tween® 20 to remove particles which were not coupled to the plate.

Then, 200µℓ of 95% ethanol was added to each well of the plate and fluorescent particles coupled to the plate were dissolved. Thereafter, dose of fluorescence of the respective wells were measured by using a fluorescent microplate reader with which a standard curve was obtained. This example may be advantageous in that it does not require any image processing technique necessary in the preceding examples.

Evaluation of antigen-antibody reaction to be performed by the standard curves will be clear for those skilled in the art.

As described hereinbefore, according to the present invention, it becomes possible to measure as small amount of antigen or antibody as 250 pg/mℓ which can not be measured by the conventional nephelometric measuring method. Further, since, in the present invention, the particle size of latex is very small, the reaction speed is very high necessarily, and there is no noise problem due to coexisting substances. Further, since, in the present invention, only the coupled fluorescent particles are picked up, it is possible to amplify a signal therefrom with a high amplification factor, resulting in a highly sensitive immunological measurement.

Although, according to the present method, a sample of low concentration can be measured, resulting in high sensitivity, it may be possible to shorten the measuring time considerably if a sample of as high concentration as in the conventional case can be used.

## Claims

1. A quantitative immunological measuring method comprising the steps of:
a) preparing a fluorescent particle reagent carrying antibody or antigen and having a mean size of not more than 0.3 µm;
b) preparing a solid phase reagent carrying antibody or antigen;
c) pouring the fluorescent particle reagent and a sample which is suspected to contain an antigen or antibody corresponding to the antibodies or antigens of said reagents to the solid phase reagent and allowing an antigen-antibody reaction to occur; and
d) quantitatively measuring those fluorescent particles coupled to the solid phase reagent.

2. The method of claim 1, wherein the quantitative measuring step comprises the step of removing those particles not coupled to said solid phase reagent.

3. The method of claim 2, further comprising the steps of dissolving the particles coupled to the solid phase reagent and measuring the dose thereof.

4. The method of claim 1, wherein the quantitative measuring step comprises the steps of:
a) obtaining particle patterns successively in time;
b) comparing the patterns to pick-up stable fluorescent particles therein; and
c) counting the stable fluorescent particles.

5. The method of anyone of claims 1 to 4, wherein the solid phase reagent comprises a plate having a plurality of wells.

6. An apparatus suitable for carring out the immunological measuring method of claim 1 which comprises:
- a flat-bottomed plate serving as a solid phase reagent carrying antibody or antigen at its surface;
- a fluorescent microscope;
- a TV camera for picking up a fluorescent particle image or a pattern of fluorescent particles through said fluorescent microscope; and
- an image processor for processing the fluorescent particle image or pattern of fluorescent particles obtained by said TV camera.

7. The apparatus of claim 6, further comprising a microcomputer for digitizing the fluorescent particle image or pattern processed by said image processor.

8. The apparatus of claims 6 or 7, further comprising a UV radiation source for emitting excitation light.

9. The apparatus of any one of claims 6 to 8, wherein the plate has a plurality of wells.

## Patentansprüche

1. Quantitative immunologische Meßmethode, umfassend die folgenden Schritte:
a) Herstellen eines fluoreszierende Teilchen-Reagens, das Antikörper oder Antigen trägt und eine mittlere Größe von nicht mehr als 0,3 µm hat;
b) Herstellen eines Festphasen-Reagens, das Antikörper oder Antigen trägt;
c) Gießen des fluoreszierende Teilchen-Reagens und einer Probe, von der angenommen wird, daß sie Antigen oder Antikörper enthält, die den Antikörpern oder Antigenen der Reagentien entsprechen, zu dem Festphasen-Reagens und Ablaufenlassen einer Antigen-Antikörper-Reaktion; und
d) quantitatives Messen jener fluoreszierenden Teilchen, die an das Festphasen-Reagens gekoppelt sind.

2. Methode nach Anspruch 1, worin der quantitative Meßschritt den Schritt des Entfernens jener Teilchen umfaßt, welche nicht an das Festphasen-Reagens gekoppelt sind.

3. Methode nach Anspruch 2, welches außerdem die Schritte des Auflösens der an das Festphasen-Reagens gekoppelten Teilchen und das Messen ihrer Dosis umfaßt.

4. Methode nach Anspruch 1, worin der quantitative Meßschritt die folgenden Schritte umfaßt:
a) Erhalten von Teilchenmustern in zeitlicher Abfolge;
b) Vergleichen der Muster, um stabile fluoreszierende Teilchen darin aufzunehmen; und
c) Zählen der stabilen fluoreszierenden Teilchen.

5. Methode nach einem der Ansprüche 1 bis 4, worin das Festphasen-Reagens eine Platte mit mehreren Vertiefungen umfaßt.

6. Vorrichtung, die zur Durchführung der immunologischen Meßmethode nach Anspruch 1 geeignet ist, welche umfaßt:
- eine flachbödige Platte, die als Festphasen-Reagens dient und Antikörper oder Antigen an ihrer Oberfläche trägt;
- ein Fluoreszenz-Mikroskop;
- eine TV-Kamera zum Aufnehmen eines Bildes oder Musters von fluoreszierenden Teilchen durch das Fluoreszenz-Mikroskop; und
- einen Bildprozessor zum Verarbeiten des mit der TV-Kamera erhaltenen Bildes oder Musters von fluoreszierenden Teilchen.

7. Vorrichtung nach Anspruch 6, welche außerdem einen Mikrocomputer zum Digitalisieren des von dem Bildprozessor verarbeiteten Bildes oder Musters von fluoreszierenden Teilchen umfaßt.

8. Vorrichtung nach Anspruch 6 oder 7, welche außerdem eine UV-Strahlungsquelle zum Emittieren von Anregungslicht umfaßt.

9. Vorrichtung nach einem der Ansprüche 6 bis 8, worin die Platte mehrere Vertiefungen aufweist.

## Revendications

1. Méthode de mesurage immunologique quantitatif,comprenant les étapes consistant à
(a) préparer un réactif particulaire fluorescent por - tant de l'anticorps ou de l'antigène et ayant une taille moyenne non supérieure à 0,3 um;
(b) préparer un réactif en phase solide portant de l'anticorps ou de l'antigène;
(c) verser le réactif particulaire fluorescent et un échantillon soupçonné contenir un antigène ou un anticorps correspondant aux anticorps ou antigènes desdits réactifs,sur le réactif en phase solide et laisser la réaction antigène-anti - corps se produire ; et
(d) mesurer quantitativement les particules fluorescentes couplées au réactif en phase solide .

2. Méthode selon la revendication 1 ,dans laquelle l'étape de mesurage quantitatif comprend l'étape d'enlèvement des particules non couplées audit réactif en phase solide.

3. Méthode selon la revendication 2 ,comprenant en outre les étapes de dissolution des particules couplées au réactif en phase solide et le mesurage de leur dose .

4. Méthode selon la revendication 1 ,dans laquelle l'étape de mesurage quantitatif comprend les étapes consistant à
(a) obtenir successivement dans le temps des tableaux ou schémas de particules ;
(b) comparer les tableaux pour y prélever des particules fluorescentes stables ; et
(c) compter les particules fluorescentes stables .

5. Méthode selon l'une quelconque des revendications là 4 ,dans lequel le réactif en phase solide comprend une plaque comportant plusieurs creux .

6. Appareil convenant pour mettre en oeuvre la méthode de mesurage immunologique de la revendication 1 ,qui com - prend :
- une plaque à fond plat servant de réactif en phase solide portant de l'anticorps ou de l'antigène à sa surface;
- un microscope à fluorescence;
- une caméra de télévision pour saisir ou prélever une image de particules fluorescentes ou un schéma ou tableau de particules fluorescentes à l'aide dudit microscope à fluorescence ; et
- un processeur d'image pour traiter l'image des particules fluorescentes ou le tableau ou schéma de particules fluorescentes obtenu(e) par ladite caméra de télévision .

7. Appareil selon la revendication 6 , comprenant en outre un micro-ordinateur pour numériser l'image des particules fluorescentes ou le tableau ou schéma traité par ledit processeur d'image .

8. Appareil selon les revendications 6 ou 7 ,comprenant en outre une source de rayonnement ultra-violet pour émettre de la lumière excitatrice .

9. Appareil selon l'une quelconque des revendications 6 à 8 , dans lequel la plaque comporte plusieurs creux .
